# EUROPEAN PATENT APPLICATION

(11) **EP 0 672 415 A1**
(43) Date of publication of application: **20.09.1995**
(21) Application number: 95102299.5
(22) Date of filing: 17.02.1995
(51) Int. Cl.: A61K 31/44, A61K 31/445, A61K 31/495

(54) **Organopathy preventing, curing or ameliorating agent**

(30) Priority: 22.02.1994 JP 46552/94; 16.11.1994 JP 282310/94
(71) Applicant: Eisai Co., Ltd., Tokyo (JP)
(72) Inventor: Takada, Yasutsugu, Kastorm Rakuhoku 707, Kyoto-shi, Kyoto (JP)
(74) Representative: Hansen, Bernd, Dr. Dipl.-Chem.

(57) **Abstract**

To provide an agent for preventing, curing or ameliorating organopathy attributed to reperfusion, ischemia or blood flow disorder in the organs such as liver, kidneys, heart, lungs, pancreas, blood vessels and small intestine, especially, those caused by transplantation and preservation of the organ and by blood reperfusion after transplantation and those occurring after surgical operation. An agent for preventing, curing or ameliorating organopathy of, for example, the liver, kidneys, heart, lungs, pancreas, blood vessels or small intestine, attributed to reperfusion, ischemia or blood flow disorder, comprising as an active ingredient a glycerol derivative represented by the following general formula or a pharmacologically acceptable salt thereof:

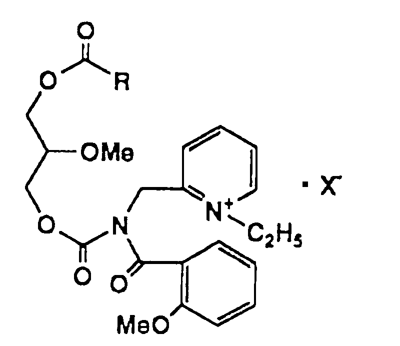

## Description

### [Field of Industrial Application]

The present invention relates to an agent for preventing, curing or ameliorating organopathy attributed to reperfusion, ischemia or blood flow disorder in the organs such as the liver, kidneys, heart, lungs, pancreas, blood vessels and small intestine, especially, those caused by transplantation and preservation of the organ and by blood reperfusion after transplantation and those occurring after surgical operation.

### [Prior arts]

In recent years, organ transplantations have become extensively carried out as surgical treatments of various types of organ insufficiency. In the organ transplantation, the organ is in ischemic condition during the period in which the organ is extirpated from an individual who supplies it (hereinafter referred to as "donor") and transplanted into another individual who receives it (hereinafter referred to as "recipient") until the blood is reperfused. The organopathy occurring in the process of the preservation of the organ under ischemic condition (hereinafter referred to as "preservation") and the blood reperfusion after transplantation (hereinafter referred to as "reperfusion") is one of the most crucial problems of the organ transplantation, which influences the take of the organ after transplantation and the results of surgical operation. Under these circumstances, an agent for preventing, curing or ameliorating organopathy attributed to preservation and reperfusion which is highly useful in the organ transplantation has been demanded.

For example, in hepatectomy, the hepatic blood circulation is blocked in order to reduce the amount of the hemorrhage. In the blocked blood circulation, often, a local blood flow disorder is caused by operative manipulations such as removal and transfer of the liver. The organopathy attributed to this blood flow disorder gravely affects the postoperative prognosis. Thus, a highly useful postoperative organopathy preventing, curing or ameliorating agent has also been demanded in the art.

Although the mechanism of the onset of the organopathy attributed to preservation, reperfusion, ischemia or blood flow disorder has not yet completely been elucidated, it has been believed that various chemotransmitters such as active oxygen, cytokine, protease, eicosanoid and platelet activating factor (PAF) are associated therewith.

For example, Surgery, 102(5), 821-827, 1987 describes that antioxidative coensyme Q₁₀ is effective in the prevention of organopathy in a rat liver transplantation experiment.

Further, Transplant., 50(1), 14-20, 1990 describes that any of the following drugs:
(1) nisoldipine which is a calcium antagonist,
(2) a mixture of diisopropyl fluorophosphate, phenylmethylsulfonyl fluoride, pepstatin A and leupeptin which are protease inhibitors, and
(3) a mixture of the drugs (1) and (2) is effective in the prevention of organopathy in a rat liver transplantation experiment.

Still further, U.S. Patent No. 5,002,965 describes that ginkgolidie of plant origin which is a cyclic compound having 20 carbon atoms is effective in the prevention of transplantation organopathy.

With respect to the effect of the coenzyme Q₁₀ described in the Surgery, 102(5), 821-827, 1987, both of the group in which the coenzyme was intravenously administered in an amount of 10 mg/kg to the donor rats prior to the surgical operation and the livers were left in ischemic condition for 60 min and transplanted and the group in which, without administering to the donor rats, the livers were left in ischemic condition for 60 min and transplanted into the recipient rats to which the coenzyme had been intravenously administered in the same amount to the recipient rats prior to the surgical operation, exhibited a one-week survival rate of about 50% (as compared with 0% of the untreated group).

However, because the coenzyme Q₁₀ is an oily substance, there is a limitation in concentration in the preparation of an injection for intravenous administration. Thus, it has been unfeasible to increase the concentration of the coenzyme Q₁₀ or the amount of administered coenzyme Q₁₀ for improving the survival rate after the surgical operation.

On the other hand, each group in which nisoldipine, a mixture of leupeptin and other protease inhibitors or a mixture of nisoldipine and the above mixture as described in the Transplant., 50(1), 14-20, 1990 was added to the donor preserving fluid exhibited a postoperative survival rate extended to about 10 - 20 days (as compared with about 1 - 2 days of the untreated group).

However, the leupeptin and pepstatin A produced by microorganisms and the phenylmethylsulfonyl fluoride being a synthetic compound are not certified drugs, and their safety in the clinical application is quite dubious. The diisopropyl fluorophosphate is unfavorable because it is highly toxic and directly affects the organ function in the transplantation, although it is in clinical use as a cholinergic (parasympathomimetic) miotic.

The ginkgolidie described in the U.S. Patent No. 5,002,965 belongs to a series of compounds extracted from plants. However, it is also not a certified drug, and its safety in the clinical application is quite dubious.

### [Description of the Invention]

Under these circumstances an agent for preventing, curing or ameliorating organopathy attributed to preservation, reperfusion, ischemia or blood flow disorder which is highly safe and highly useful (in the survival rate and the preventive and curative effects) has been demanded.

Therefore, the inventors have made intensive studies on the compounds meeting the above requirements. As a result, it has been found that the above glycerol derivative can attain the desired goal as an organopathy preventing, curing and ameliorating agent. The present invention has been completed on the basis of the above finding. Accordingly, an object of the present invention is to provide an agent which can prevent, cure or ameliorate organopathy occurring in the stages of preservation of the organ under ischemic condition, blood reperfusion after the transplantation and blood flow disorder caused by the surgical operation and is thus highly useful in clinical application.

The glycerol derivative of the present invention is a compound described in Japanese Patent Laid-Open No. 131467/1990, which is an agent for preventing and curing DIC, shock, allergy, acute pancreatitis and cerebral twitch at the time of subarachnoid hemorrhage and has an antiplatelet activating factor (PAF) activity. Further study by the inventors has led to an unexpected finding that the glycerol derivative also has an organopathy preventing, curing or ameliorating activity, on the basis of which the present invention has been completed.

The glycerol derivative of the present invention is represented by the following general formula: 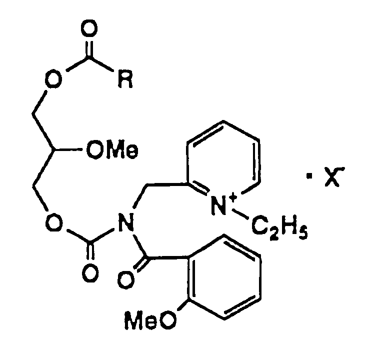
wherein
R represents a group represented by the following chemical formula: 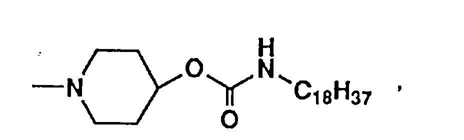
a group represented by the following chemical formula: 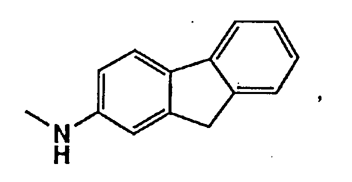
a group represented by the following chemical formula: 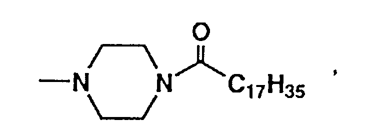
a group represented by the following chemical formula: 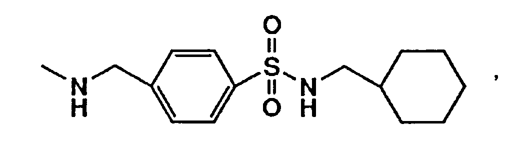
a group represented by the following chemical formula: 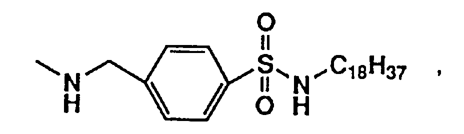
or a group represented by the following chemical formula: 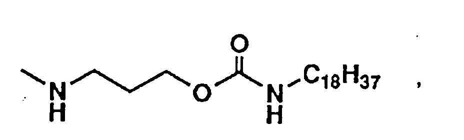
and X represents an atom or atomic group convertible to an anion.

The invention moreover provides a method for preventing, treating, curing or ameliorating an organopathy attributed to reperfusion, ischemia or blood flow disorder by administering a pharmacologically effective amount of the above defined glycerol derivative or a pharmacologically acceptable salt thereof, to a subject suffering from the organopathy.

It is preferable that the organ is at least one member selected from the group consisting of the liver, kidneys, heart, lungs, pancreas, blood vessels and small intestine. The organ may be at least one member selected from the group consisting of the liver, kidneys, heart, lungs, pancreas and small intestine and the organopathy is transplantation or postoperative transplantation.

It is preferable that the glycerol derivative is 1-ethyl-2-[N-(2-methoxy)benzoyl-N-{2-methoxy-3-(4-octadecylcarbamoyloxy)piperidinocarbonyloxypropoxy}carbonyl]aminomethylpyridinium chloride.

The invention, in addition, provides use of the glycerol derivative as defined above or a pharmacologically acceptable salt thereof for manufacturing a medicine being effective for preventing, treating, curing or ameliorating an organopathy attributed to reperfusion, ischemia or blood flow disorder; a pharmaceutical agent comprising the glycerol derivative as defined above or a pharmacologically acceptable salt thereof for preventing, treating, curing or ameliorating an organopathy attributed to reperfusion, ischemia or blood flow disorder; and a pharmaceutical composition comprising a pharmacologically effective amount of the glycerol derivative as defined above or a pharmacologically acceptable salt thereof and a pharmacologically acceptable carrier.

Examples of the glycerol derivatives according to the present invention include the following compounds:
(1) 1-Ethyl-2-[N-(2-methoxy)benzoyl-N-{2-methoxy-3-(4-octadecylcarbamoyloxy)piperidinocarbonyloxypropoxy}carbonyl]aminomethylpyridinium chloride,
(2) 1-ethyl-2-[N-{3-(2-fluorenamino)carbonyloxy-2-methoxypropoxy}carbonyl-N-(2-methoxy)benzoyl]aminomethylpyridinium chloride,
(3) 1-ethyl-2-[N-(2-methoxy)benzoyl-N-{2-methoxy-3-(4-stearoylpiperazinocarbonyl)oxypropoxy}carbonyl]aminomethylpyridinium iodide,
(4) 1-ethyl-2-[N-{3-(4-cyclohexylmethylsulfamoyl)benzylcarbamoyloxy-2-methoxypropoxy}carbonyl-N-(2-methoxy)benzoyl]aminomethylpyridinium chloride,
(5) 1-ethyl-2-[N-(2-methoxy)benzoyl-N-{2-methoxy-3-(4-octadecylsulfamoyl)benzylcarbamoyloxypropoxy}carbonyl]aminomethylpyridinium chloride, and
(6) 1-ethyl-2-[N-(2-methoxy)benzoyl-N-{2-methoxy-3-(3-octadecylcarbamoyloxy)propylcarbamoyloxy}propoxycarbonyl]aminomethylpyridinium chloride.

Of the above compounds, 1-ethyl-2-[N-(2-methoxy)benzoyl-N-{2-methoxy-3-(4-octadecylcarbamoyloxy) piperidinocarbonyloxypropoxy}carbonyl]aminomethylpyridinium chloride is preferred.

The present invention provides an organopathy preventing, curing or ameliorating agent comprising the above glycerol derivative compound or a pharmacologically acceptable salt thereof as an active ingredient. The pharmacologically acceptable salt of the present invention depends on the type of X of the chemical formula representing the above glycerol derivative. In the chemical formula, X represents an atom or atomic group convertible to an anion. In particular, it represents counter ions of quaternary ammonium salts, examples of which include chloride, bromide, iodide, sulfate, nitrate, phosphate, perchlorate, methanesulfonate, ethanesulfonate, benzenesulfonate, p-toluenesulfonate, camphorsulfonate and hydroxide ions. These ions are not particularly limited, although chloride ion is preferred.

The glycerol derivative compounds of the present invention can be produced by the processes described in Examples 2, 11, 12, 13, 14 and 15 of the above Japanese Patent Laid-Open No. 131467/1990.

### [Brief Description of the Drawings]

[Fig. 1] Graph showing the changes of AKBR values of treated and untreated groups with the passage of time (values expressed by average ± standard error).

[Fig. 2] Graph showing the changes of arterial blood lactate level values of treated and untreated groups with the passage of time (values expressed by average ± standard error).

[Fig. 3] Graph showing the changes of hematocrit values of treated and untreated groups with the passage of time (values expressed by average ± standard error).

[Fig. 4] Graph showing the changes of platelet count values of treated and untreated groups with the passage of time (values expressed by average ± standard error).

[Fig. 5] Graph showing the changes of leukocyte count values of treated and untreated groups with the passage of time (values expressed by average ± standard error).

[Fig. 6] Graph showing the changes of GOT values of treated and untreated groups with the passage of time (values expressed by average ± standard error).

[Fig. 7] Graph showing the changes of GPT values of treated and untreated groups with the passage of time (values expressed by average ± standard error).

### [Pharmacological Tests]

The results of an acute toxicity test are shown below of 1-ethyl-2-[N-(2-methoxy)benzoyl-N-{2-methoxy-3-(4-octadecylcarbamoyloxy)piperidinocarbonyloxypropoxy}carbonyl]aminomethylpyridinium chloride [hereinafter referred to as "compound (I)"] as a representative example of the glycerol derivative of the present invention.

### [Acute Toxicity Test]

A single administration toxicity test was carried out by effecting intravenous injections (medium: physiological saline) to a group consisting of five male and five female 7 to 8-weeks-old Slc:SD rats and a group consisting of five male and five female 7 to 8-weeks-old Slc:ICR mice. The obtained LD₅₀ values are summarized in the following Table.

### [Table 1]

### Acute toxicity (LD₅₀: mg/kg) of 1-ethyl-2-[N-(2-methoxy)benzoyl-N-{2-methoxy-3-(4-octadecylcarbamoyloxy)piperidinocarbonyloxypropoxy}carbonyl]aminomethylpyridinium chloride [compound (I)]

| Administrat ion Path | Mouse | | Rat | |
|---|---|---|---|---|
| | Male | Female | Male | Female |
| Intravenous Injection | 46.8 mg/kg | 50.0 mg/kg | 18.2 mg/kg | 22.8 mg/kg |

The above LD₅₀ values are at least as large as about 50 times the clinical dose in intravenous injection, thereby attesting to extremely high safety.

A test example for examining the function of the compound (I) as a representative compound for preventing or ameliorating transplantation organopathy with respect to a porcine liver transplantation model will be set forth below in order to demonstrate the effect of the present invention.

### (Experimental Method)

### (1) Experimental Design

A model was employed in which swine each weighing 30 to 40 kg were used, the livers were extirpated therefrom and preserved at low temperature for 8 hr, and sympatric liver transplantation was carried out. More specifically, the swine were starved for 24 hr, and randomly divided into two groups. Group 1 consisted of 9 donors and 9 recipients, which were treated with the above compound of the present invention. In the surgical operation of the donors, the compound (I) was added to the perfusate and the preservation fluid (1 mg/ℓ). Further, the compound (I) was continuously intravenously administered to the recipients (0.3 mg/kg) and added, in the surgical operation thereof, to the washing fluid for washing away the preservation fluid from the interior of each of the transplant livers (1 mg/ℓ).

Group 2 as a control group not treated with the compound of the present invention consisted of 9 donors and 9 recipients, for which the same continuous intravenous administration was carried out with the use of the same fluids as for Group 1, except that the compound (I) was not added thereto. A University of Wisconsin (hereinafter referred to as "UW") fluid prepared in a simplified manner was used for the perfusion and preservation of the transplant livers. The fluid was one obtained by omitting hydroxyethylstarch from the composition of the UW fluid customarily employed in the preservation of livers.

### (2) Operation of Donors

For each of the donors, premedication with ketamine (10 mg/kg) was conducted, and the auricular vein was cannulated to initiate the administration of physiological saline. Ketamine (5 mg/kg, i.v.) and pancronium (0.2 mg/kg, i.v.) were administered, and thereafter the airway was secured. The donor under surgical operation was kept anesthetized by inhalation of 3 ℓ of oxygen, 4 ℓ of laughing gas and 1.5% ethrane.

The subhepatic inferior vena cava was stripped till a site below the renal vein, the common bile duct was ligated and excised, and the portal vein and the hepatic artery were stripped and exposed. Heparin (10,000 IU) was intravenously injected, and thereafter the subrenal ventral aorta was cannulated. The splenic vein was ligated, and cannulation via the superior mesenteric vein was effected. Immediately thereafter, the aorta vas cross-clamped at the site of the diaphragm, and hepatic perfusion with the UW fluid prepared in a simplified manner was initiated via the aorta (500 ml) and the superior mesenteric vein (1500 ml). The gallbladder was dissected, and washed with physiological saline. The resultant liver was taken out, and immersed in a UW fluid bath. The right adrenal was excised from the inferior vena cava, and the cuff was tied with the portal vein and the subhepatic inferior vena cava. The transplant liver was preserved at 4°C for 8 hr.

### (3) Operation of Recipients

Each of the recipient swine was also anesthetized in the same manner as described above with respect to the surgical operation of the donors. The right carotid artery and the right subclavian vein were cannulated for blood pressure monitoring and blood sampling and for intravenous injection, respectively. The abdomen was cut open, and the liver was detached and removed. During the ahepatic period, the blood from the portal vein was passively bypassed through a shunt tube inserted into the portal vein and the right external jugular vein. The end-to-end anastomosis of the transplant liver was carried out with the suprahepatic inferior vena cava (5-0 Prolene, running suture), portal vein (cuff technique), subhepatic inferior vena cava (cuff technique) and hepatic artery (7-0 Prolene, intermittent suture) in this order to thereby reconstruct the liver, so that a sympatric transplantation was achieved. After the completion of the anastomosis with the portal vein, the transplant liver was reperfused with the blood from the portal vein.

The bile duct was drained outside the body through a biliary external fistula tube.

Just before the anastomosis with the portal vein, the transplant liver was perfused with lactated Ringer's solution (500 ml) containing dextrose (5 wt.%), mannitol (1.25 wt.%) and sodium bicarbonate (0.21 wt.%) to thereby remove the preservation fluid. The washing fluid was held at 30 to 37°C. Within 3 min of the hot washing, the transplant liver was reperfused.

During the ahepatic period starting with the initiation of the surgical operation, physiological saline (500 ml) containing dextrose (2.5 wt.%) was administered through the auricular vein. For the Group 1, the compound (I) (0.3 mg/kg) was mixed in the saline solution. During the ahepatic period, 500 to 750 ml of physiological saline containing sodium bicarbonate (0.21 wt.%) was rapidly injected in order to prevent blood pressure lowering. Sodium bicarbonate (1.05 g) and calcium gluconate (0.1 g) were intravenously administered prior to the reperfusion of the transplant liver. During the surgical operation, no transfusion was conducted, and a total of 1.5 to 2.0 ℓ of infusion solution was administered. For the first 12 hr after the surgical operation, 0.5 to 1.0 ℓ of 5% dextrose solution was infused. Sodium bicarbonate was used for correction of metabolic acidosis according to necessity. No immunosuppressive agent was used at all.

### (4) Tested Compound

The compound (I) soluble in both water and physiological saline was dissolved in physiological saline to prepare a solution of the compound (I) (1.5 mg/ml) prior to use.

### (5) Test Items

The 3-hydroxybutyrate and acetoacetate levels of arterial blood were measured at the time of the cannulation of the right carotid artery (prior value) and 1, 2, 4, and 12 hr after the reperfusion, and their ratios (acetoacetate/3-hydroxybutyrate, hereinafter referred to as "AKBR") were calculated. The measurements for AKBR were performed with the use of Ketorex Bit and Keto-340 (both produced by Sanwa Kagaku Kenkyusho Co., Ltd.). The prior value and the values at 2, 4 and 12 hr after the reperfusion were measured of the lactate level and hemogram (hematocrit, platelet count and leukocyte count) of the arterial blood. GOT and GPT were also measured at the same points, and further measured 1, 2 and 3 days after the surgical operation.

### (6) Histologic Condition

Cuneate hepatic tissue was harvested 1 hr after the reperfusion (just before abdominal suture). For optical microscope observation, the specimen was immobilized with 10% formaldehyde and stained with hematoxylin-eosin or periodic acid schiff stain.

### (7) Statistical Analysis

Test data were expressed by "average ± standard error". Average differences were compared between the Group 1 and the Group 2 by the use of Student's t-test (unpaired). The survival rate difference between the two groups 12 hr after the reperfusion or later was evaluated by the use of chi-square test. When the P value was less than 0.05, it was judged that there was a significant difference.

### (Results)

The postoperative course of each of the groups is shown below.

**[Table 2]**

| Course After Transplantation | | | |
|---|---|---|---|
| Group | Individual No. | Period of Survival | Case of Death |
| 1 Treated Group | 1 | 4 days | air embolism |
| | 2 | 5 days | unclear |
| | 3 | 6 days | ileus |
| | 4 | 6 days | hemorrhagic gastric ulcer |
| | 5 | 7 days | hemorrhagic gastric ulcer |
| | 6 | 7 days or more | killed for autopsy |
| | 7 | 7 days or more | killed for autopsy |
| | 8 | 7 days or more | killed for autopsy |
| | 9 | 7 days or more | killed for autopsy |
| 2 Untreated Group | 10 | 12 hours | metabolic acidosis |
| | 11 | 12 hours | metabolic acidosis |
| | 12 | 12 hours | metabolic acidosis |
| | 13 | 12 hours | metabolic acidosis |
| | 14 | 2 days | hemorrhagic gastric ulcer |
| | 15 | 6 days | hemorrhagic gastric ulcer |
| | 16 | 7 days or more | killed for autopsy |
| | 17 | 7 days or more | killed for autopsy |
| | 18 | 7 days or more | killed for autopsy |

All the recipient swine of the two groups recovered after the surgical operation, and were decannulated within 4 hr of the reperfusion. However, in the Group 2 (untreated group), 4 of the 9 swine died within 4 to 12 hr of the surgical operation. In contrast, in the Group 1 (treated group), all of the 9 swine survived for at least 4 days (see Table 1). The 12-hr or longer survinal rate of the Group 1 was 100% (9/9), whereas that of the Group 2 was 56% (5/9).

The AKBR is a parameter indicative of the performance of the hepatocytes, by which it is feasible to trace the change of the condition of the liver with the passage of time. Regularly (in normal condition), it is held at 1.0 or higher. However, when the liver is shocked or otherwise brought into disorder, it is decreased. Most of the livers die when their AKBR values reach 0.4 or less (see pages 141-149 of "Rinsho-i no tame no hando-bukku (Handbook for Clinician), Shock Q&A", edited by Masanobu Tamakuma, Ryu Ogawa and Naoki Aikawa, supervised by Hideo Yamamura and published by Medical Review).

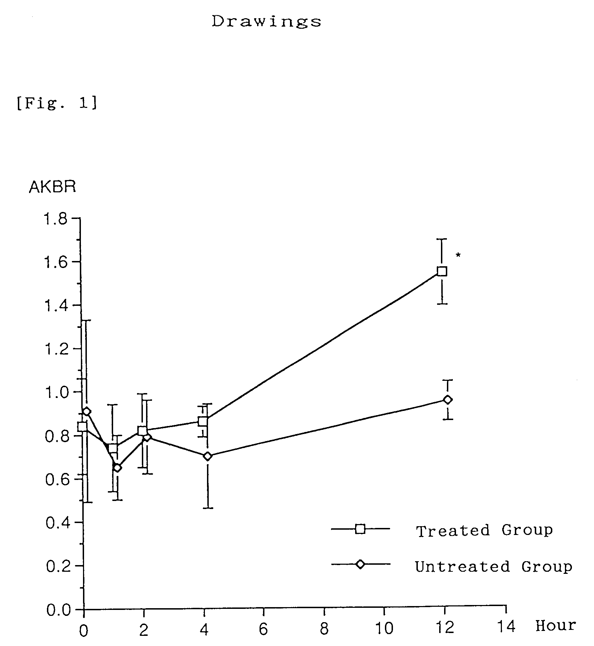

Fig. 1 shows the change of AKBR exhibited in this experiment.

During 4 hr after the reperfusion, the AKBR of the Group 1 was only slightly higher than that of the Group 2. However, the AKBR of the Group 1 rapidly increased thereafter and recovered to the normal condition level 12 hr later, thereby having a significantly high value as compared with the AKBR of the Group 2 (p<0.05).

This demonstrates that the administration of the compound (I) to the donor and the recipient prevents or ameliorates the organopathy attributed to preservation and reperfusion in transplantation.

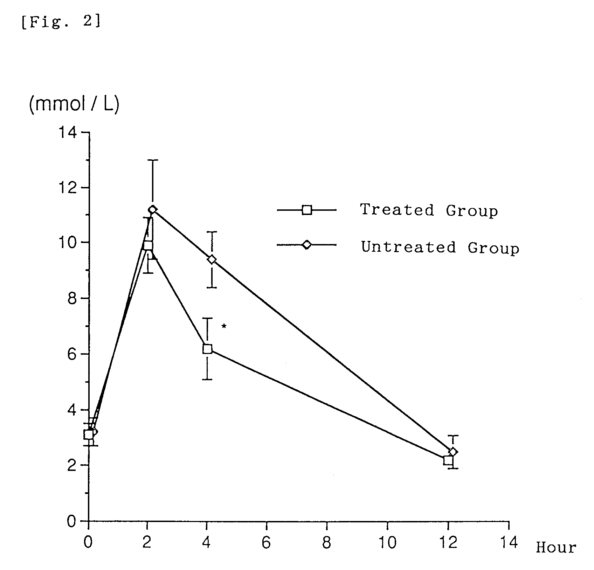

Fig. 2 shows the change of lactate level of arterial blood.

The lactate levels of the Groups 1 and 2 increased to 9.9 ± 1.0 and 11.2 ± 1.8 mmol/ℓ, respectively, 2 hr after the reperfusion. However, 4 hr after the reperfusion, the lactate level of the Group 1 rapidly decreased to 6.2 ± 1.1 mmol/ℓ (p<0.05), whereas that of the Group 2 decreased only to 9.4 ± 1.0 mmol/ℓ. The lactate levels of both of the groups recovered to the initial level 12 hr after the reperfusion.

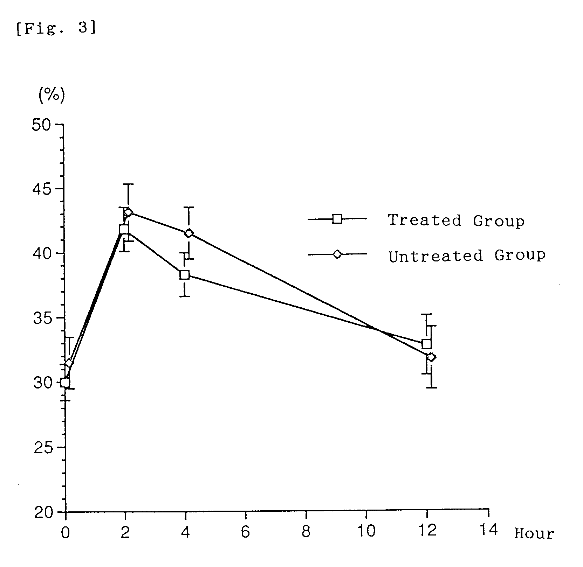

The changes of hematocrit, platelet count and leukocyte count values of the two groups are shown in Figs. 3, 4 and 5, respectively.

The hematocrit values of the two groups rapidly increased 2 hr after the reperfusion, and gradually decreased thereafter (see Fig. 3).

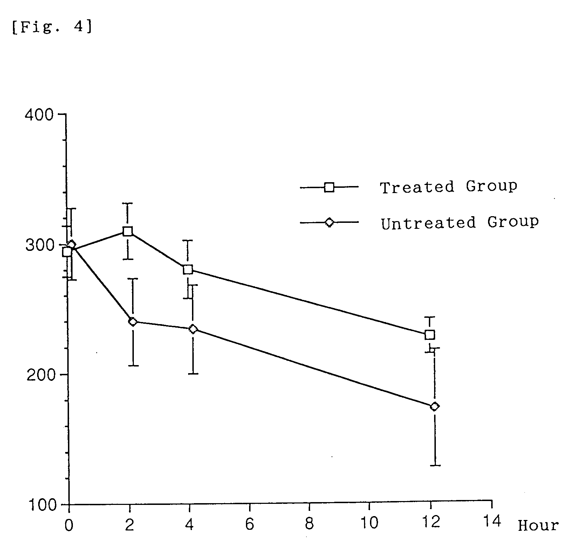

The platelet count of the Group 1 exhibited a slight increase from 294.4 ± 19.8 (prior value, ×10³/µℓ) to 309.8 ± 21.6 (2 hr later, ×10³/µℓ), and thereafter decreased to 227.8 ± 13.4 (12 hr later, ×10³/µℓ). On the other hand, the platelet count of the Group 2 decreased from 300.1 ± 27.5 (prior value, ×10³/µℓ) to 240.0 ± 33.4 (2 hr later, ×10³/µℓ), and thereafter further decreased to 172.6 ± 45.1 (12 hr later, ×10³/µℓ). Although no significant difference was recognized between the two groups, the platelet count decrease was less in the Group 1 treated with the compound of the present invention (see Fig. 4).

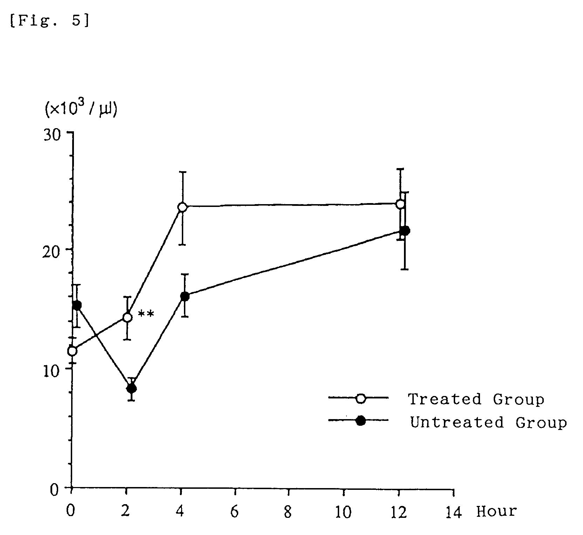

The leukocyte count of the Group 1 exhibited a slight increase from 11.5 ± 1.1 (prior value, ×10³/µℓ) to 14.3 ± 1.8 (2 hr later, ×10³/µℓ), and thereafter further increased. On the other hand, the leukocyte count of the Group 2 decreased from 15.3 ± 1.8 (prior value, ×10³/µℓ) to 8.3 ± 0.9 (2 hr later, ×10³/µℓ), and thereafter increased. The leukocyte count exhibited 2 hr after the reperfusion was significantly higher in the Group 1 than in the Group 2 (p<0.01, see Fig. 5).

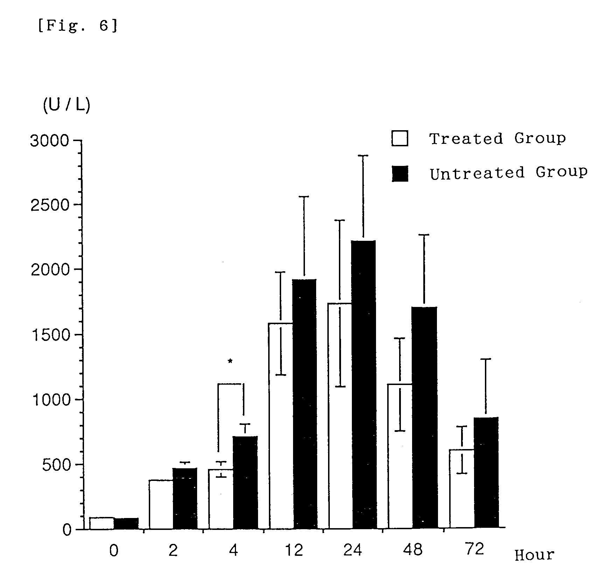

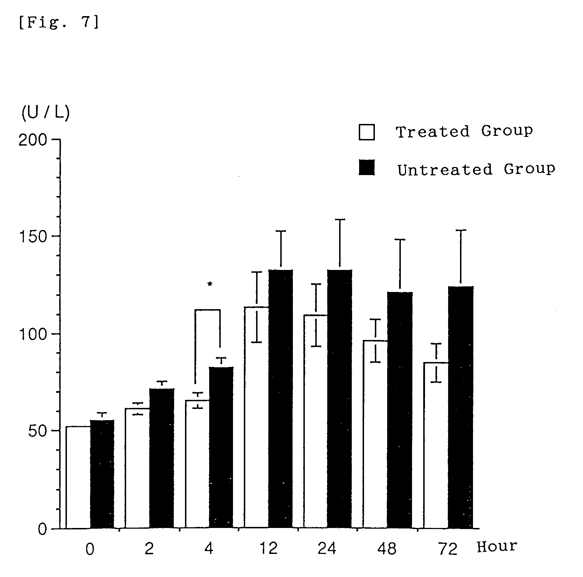

The postoperative changes of GOT and GPT are shown in Figs. 6 and 7, respectively.

GOT and GPT are enzymes leaking from impaired hepatocytes. The GOT levels of the two groups both increased within a period of 24 hr after the reperfusion. The GOT level exhibited 4 hr after the reperfusion was significantly higher in the Group 2 than in the Group 1 (Group 1: 712 ± 97, Group 2: 461 ± 59 U/ℓ, p<0.05). Thereafter, also, the GOT level was higher in the Group 2 than in the Group 1 (see Fig. 6).

The GPT level also was higher in the Group 2 than in the Group 1, and a significant difference was recognized 4 hr after the reperfusion (Group 1: 65 ± 4 U/ℓ, Group 2: 82 ± 5 U/ℓ, p<0.05)(see Fig. 7).

Another pharacological tests were conducted as follows:

### (Experimental Method)

### (1) Experimental design

Orthotopic liver transplantations following 8 hour cold preservation were performed. Large-White pigs weighing 35-40 Kg were used after 24 hours starvation. Pigs were randomly divided into 2 groups. In Group 1, the PAF antagonist treatment group, consisting of 9 donors and 9 recipients, the PAF antagonist the compound (I) was given in the solutions (1mg/L) for flush-out and preservation at donor operation. It was also administered into recipients (0.3mg/Kg body weight) as an infusion and given in the solution (1mg/L) for rinsing before reperfusion of the graft (RPF) at recipient operation. Group 2, the untreated control group, consisted of 9 donors and 9 recipients that received the same solutions or intravenous infusion only without the compound (I). The solutions for flush-out and preservation of the graft liver were simplified UW solution in which hydroxyethyl starch was omitted from the original formulation of UW solution described for liver preservation, according to the reports showing that hydroxyethyl starch is not an essential component in UW solution.

### (2) Donor operation

Following premedication with ketamine (10mg/Kg i.m.), the ear vein was cannulated and saline perfusion was started. After injection of ketamine (5mg/Kg i.v.) and pancuronium (0.2mg/Kg i.v.), the animals were incubated. Anesthesia was maintained by inhalation with 3L oxygen, 4L nitrous oxide and 1.5% ethranne.
After dissection of the infrahepatic inferior vena cava to the level below the renal veins, the common bile duct was ligated and cut, and the portal vein and hepatic artery were dissected and exposed. Following intravenous injection of heparine (10,000IU), the subrenal abdominal aorta was cannulated. Immediately after the ligation of the splenic vein and the cannulation through the superior mesenteric vein, the aorta was cross-clamped at the level of the diaphragm and the flash-out of the liver with simplified UW solution (4üÄ) was started though the aorta (500ml) and the superior mesenteric vein (1,500ml). The gallbladder was incised and washed with saline. The liver was harvested and dipped in 4üÄ UW solution bath. After the right adrenal gland was resected from the inferior vena cava and cuffs were connected to the portal vein and infrahepatic inferior vena cava, the liver graft was stored at 4üÄ for 8 hours.

### (3) Recipient operation

The recipient pigs were anesthesized as described in donor operation. The right carotid artery and the right subclavian vein were then cannulated for blood pressure monitoring and blood sampling and for intravenous fluid infusion, respectively. After laparotomy, the liver was dissected free and removed. During anhepatic period, the portal blood was passively shunted by a tube between the portal vein and the right external jugular vein. The graft liver was implanted orthotopically with end-to-end anastomoses of the suprahepatic inferior vena cava (5-O Prolene, running suture), the portal vein (cuff technique), the infrahepatic inferior vena cava (cuff technique) and the hepatic artery (7-O Prolene, intermittent suture) in this order. The graft liver was reperfused with the portal blood after completion of the portal vein anastomosis. The bile duct was drained by external tube fistula.
Immediately before the anastomosis of the portal vein, to remove the preservation solution, the graft liver was flushed with 500ml of Ringers lactate containig dextrase (5g%), mannitol (1.25g%) and sodium bicarbonate (0.21g%) as previously described. This rinsing solution was kept between 30-37üÄ, because it has been suggested that a brief rinse of liver grafts with warm buffer markedly improves the hepatic microcirculation, leading to dramatic improvement in graft survival. In our operations, the duration between the warm rinsing and RPF was less than 3 miniutes by using the cuff technique in portal vein anastomosis.

Intraoperative fluid infusion was as follows; 500ml of saline containing dextrose (2.5g%) was infused through the ear vein between the begining of the operation and the anhepatic period. In Group 1, The compound (I) (0.3mg/Kg) was mixed in this solution. During the anhepatic period, 500-750ml of saline containing sodium bicarbonate (0.21g%) was rapidly infused to prevent hypotension. Prior to RPF, sodiumu bicarbonate (1.05g) and calcium gluconate (0.1g) were injected intravenously. As a total, 1.5-2L of the fluid was infused during operation without any blood transfusion. Postoperatively, 0.5-1.0L of 5% dextrose solution was infused for the first 12 hours. Sodium bicarbonate was given to correct metabolic acidosis when necessary. No immunosuppressant was administered.

### (4) Tested Compound

### The compound (I)

(1-Ethyl-2-[N-(2-methoxy)benzoyl-N-[(2R)-2-methoxy-3-( 4-octadecylcarbamoyloxy)piperidinocarbonyloxypopoxy]carbonyl]aminomethylpyridinium chloride, molecular weight 860.5) was supplied in powder form by Eisai Co.,Ltd. (Japan). It is soluble in water and saline, and a 1.5 mg/ml solution was prepared with saline immediately before use. It was reported that the compound (I) inhibits aggregation of human platelets induced by PAF, with IC50 value of 0.66nM.

### (5) Parameters

AKBR was measured at the time of cannulation in the right carotid artery (before), and at 1, 2, 4 and 12 hours after RPF. AKBR, the ratio of acetoacetate over 3-hydroxybutyrate in the arterial blood, was measured enzymatically using the Ketorex kit (Sanwa Chemicals, Nagoya, Japan) and Keto-340 (a semiautomatic spectorophotometer designed for the measurement of ketone bodies, Ihara Electric Co., Kasugai, Japan), as described previously. Lactate levels in arterial blood and blood count analyses (hematocrit, platelet and white blood cell counts) were determined before and at 2, 4, 12 hours after RPF, GOT and GPT were also measured at the same time and on 1(24 hours), 2 and 3 postoperative days (POD).

### (6) Histological findings

Liver tissues were taken by wedge resection for histological examination at 1 hour after RPF (immediately before abdominal closure). For light microscopy, specimens were fixed in 10% formalin and stained with hematoxylin-eosin (HE) or with periodic acid schiff (PAS) for electron microscopy.

### (7) Statistical Analysis

Values are expressed as meansü}SEM. The differences of the mean values were compared between Groups 1 and 2 using Student's t-test (unpaired). The differences of survival more than 12 hours after RPF between the two groups were analyzed by χ-square test. P values less than 0.05 were regarded as significant.

### (Results)

All the recipient pigs in both groups recovered from operation and could be extubated within 4hours after RPF. However, 4 out of 9 pigs in Group 2 (untreated) died between 4 and 12 hours. By contrast, all the 9 pigs in Group 1 (treated) survived more than 4 days (Table 1). Even in Group 2, the remaining pigs which could get over the acute critical phase survived more than 6 days except for one pig that died of hemorrhagic gastric ulcer on 2 POD. The survival rate more than 12 hours were 100% (9/9) in Group 1 and 56% (5/9) in Group 2 (p<0.05). Accordingly, after 12 hours, differences of the following parameters were compared between 9 survivors in Group 1 and 5 in Group 2.

Figure 1 shows the changes in AKBR. At 4 hours after RPF, AKBR showed tendency to be higher in Group 1 than in Group 2, though not significant. Afterward, AKBR in Group A rapidly increased to 1.54ü}0.15 at 12 hours and it was significantly higher than in Group 2 (0.95ü}0.09, p<0.05).

Figure 2 shows the changes in lactate level in arterial blood. At 2 hours after RPF, the lactate level increased to 9.9ü}1.0 and 11.2ü}1.8 mmol/L in Group 1 and 2, respectively, However, at 4 hours, in decreased rapidly to 6.2ü}1.1 mmol/L in Group 1 compared to 9.4ü}1.0 mmol/L in Group 2 (p<0.05). At 12 hours, it was restored to the primary levels in both groups.

Figure 3, 4 and 5 show the changes in hematocrit, platelet and white bolld cell (WBC) counts, respectively. In Figure 3, hematocrit values in both groups increased markedly at 2 hours after RPF, and then gradually decreased. In Figure 4, the platelet count in Group 2 decreased from 300.1ü}21.6 (before) to 240.0ü}33.4 (ü 103/ml) at 2 hours, and sbseqently showed further decreases. On the other hand, it slightly increased at 2 hours in Group 1, though it decreased follwingly and there was no significant difference between the 2 groups. Figure 5 demonstrates that the WBC count in Group 2 decreased from 15.3ü}1.8 (before) to 8.3ü}0.9 (ü 103/ml) at 2 hours. By contrast in Group 1, it slightly increased to 14.3ü}1.8 (ü 103/ml) at 2 hours and it was significantly more than in group 2 (p<0.01).

Afterward, it increased in both groups.

Postperative changes in GOT are shown in Figure 6. Within 24 hours after RPF, GOT continued to increase in both groups. At 4 hours, it was sinificantly higher in Group 2 than in Group 1 (712ü}97 v.s. 461ü}59 U/L, p<0.05). After that, though not significant, it remained to be higher in Group 2 than in Group 1. Also, as shown in Figure 7, there was a tendency that postoperative increase in GPT was higher in Group 2 than in Group 1, and at 4 hours, a significant difference was obtained (65ü}4 U/L in Group 1, v.s. 82ü}5 U/L in Group 2, p<0.05).

Histological examination by light microscopy (HE stain) revealed that there are some spotty or zonal necrosis of hepatocytes, a severe exudate of inflammatory cells (neutrophils) sludged in the sinusoids, and microvesicular changes of some hepatocytes in the reperfused liver of Group 2 pigs and that these findings are rarely seen in that of Group 1 pigs. PAS staining examination showed lesser content of glycogen in hepatocytes in Group 2 than in Group 1.

Results are shown in Table 4.

**Table 4**

| Survival after liver transplantation | | |
|---|---|---|
| | Survival | Cause of death |
| Group 1 (n=9) (treated) | 4 POD | Airembolism |
| | 5 POD | Unknown |
| | 6 POD | Intestinal obstruction |
| | 6 POD | Hemorrhagic gastric ulcer |
| | 7 POD | Hemorrhagic gastric ulcer |
| | >7 POD | Sacrifice# |
| | >7 POD | Sacrifice |
| | >7 POD | Sacrifice |
| | >7 POD | Sacrifice |
| Group 2 (n=9) (untreated) | <12 Hours## | Metabolic acidosis |
| | <12 Hours## | Metabolic acidosis |
| | <12 Hours## | Metabolic acidosis |
| | <12 Hours## | Metabolic acidosis |
| | 2 POD | Hemorrhagic gastric ulcer |
| | 6 POD | Hemorrhagic gastric ulcer |
| | >7 POD | Sacrifice |
| | >7 POD | Sacrifice |
| | >7 POD | Sacrifice |

| | | |
|---|---|---|
| # ; When pigs survived more than 7 POD, they were sacrificed. | | |
| ##; Survival rates more than 12 hours were 100% (9/9) in Group 1 and 56% (5/9) in Group 2 (p<0.05). | | |

Histologic study by the use of an optical microscope showed that each of the reperfused livers of the Group 2 had some punctate or cingulate hepatocyte necroses, conglobation and stagnation of hepatosinusoidal inflammatory cells (neutrophils) and vacuolar degeneration detected in some hepatocytes. These conditions were little observed in the Group 1. As a result of staining, it was found that the glycogen content of the hepatocytes in the Group 2 was smaller than that in the Group 1.

As apparent from the above toxicity test results and effect demonstrating examples, the compound of the present invention has an excellent activity of preventing or curing the organopathy attributed to preservation, reperfusion and postoperative blood flow disorder in transplantation, and hence is clinically useful as an organopathy preventing, curing or ameliorating agent.

In the use of the compound of the present invention as an agent for curing or ameliorating transplantation organopathy, although the administration path and the dose depend on the patient's condition, severity, kind of transplanted organ, age, and cardiac, hepatic and renal activities, and are not particularly limited, generally 0.1 to 100 mg, preferably 0.3 to 30 mg, still preferably 0.5 to 20 mg and further still preferably 1 to 10 mg thereof is daily administered to an adult recipient intravenously, orally, nasally, as a suppository or percutaneously. On the other hand, generally 10 mg/ml, preferably 1 mg/ml thereof is added to each of the perfusate and the preservation fluid for the donor organ.

The pharmaceutical preparation for injection can be produced by the customary procedure in which conventional pharmaceutical carriers such as a pH regulator, a buffer, a stabilizer and a solubilizing agent are added to the base, according to necessity.

A working example suitable for producing a pharmaceutical preparation for injection comprising an active ingredient of the compound (I) as a representative of the compounds according to the present invention will be described below, which should not be construed as limiting the examples of the present invention.

### [Example]

### Example 1 Pharmaceutical preparation for injection comprising 1-ethyl-2-[N-(2-methoxy)benzoyl-N-{2-methoxy-3-(4-octadecylcarbamoyloxy)piperidinocarbonyloxypropoxy}carbonyl]aminomethylpyridinium chloride

The following ingredients were dissolved in distilled water or physiological saline for injection, and the pH was regulated with citric acid. The mixture was subjected to bacterial filtration, and lyophilized, thereby obtaining a pharmaceutical preparation for injection.

**[Table 3]**

| Composition for 1 Vial (unit: mg) | |
|---|---|
| Compound (I) | 1.0 |
| Mannitol | 1.0 |
| Citric Acid | appropriate amount |

## Claims

1. Use of a glycerol derivative represented by the following formula 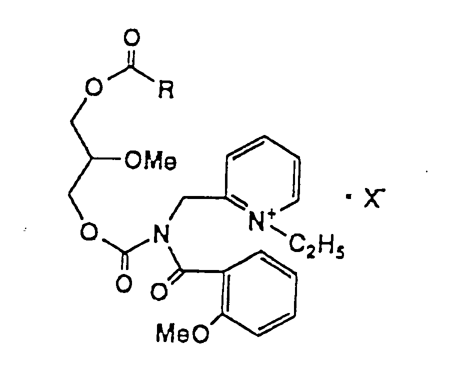 wherein
R is a group selected from the group consisting of the groups having the respective chemical formulae (2) to (7): 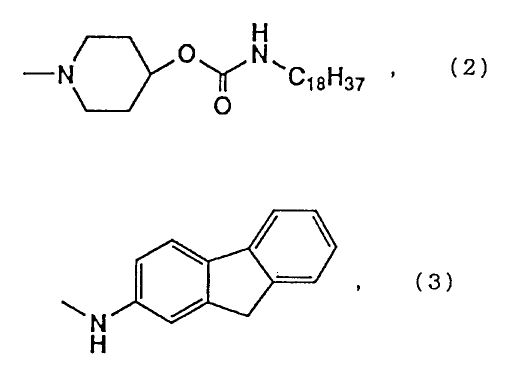 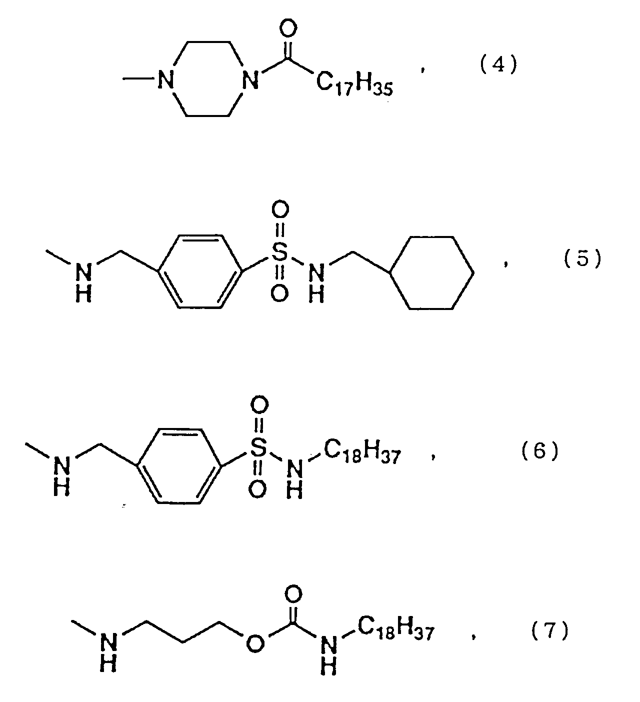 and X is an anion group of an atom or atomic group, or a pharmacologically acceptable salt thereof for manufacturing a medicine being effective for preventing, treating, curing or ameliorating an organopathy attributed to reperfusion, ischemia or blood flow disorder.

2. Use according to claim 1, for the preparation of a medicament for the treatment of organopathy of the liver, the kidneys, the heart, the lungs, the pancreas, the blood vessels or the small intestine.

3. Use according to claim 2, whereby the organopathy is transplantation or postoperative transplantation.

4. Use according to claim 1, whereby the glycerol derivative is 1-ethyl-2-[N-(2-methoxy)benzoyl-N-{2-methoxy-3-(4-octadecylcarbamoyloxy)piperidinocarbonyloxypropoxy}carbonyl]aminomethylpyridinium chloride.

5. A pharmaceutical agent comprising the glycerol derivative as defined in Claim 1 or a pharmacologically acceptable salt thereof for preventing, treating, curing or ameliorating an organopathy attributed to reperfusion, ischemia or blood flow disorder.

6. A pharmaceutical composition comprising a pharmacologically effective amount of the glycerol derivative as defined in Claim 1 or a pharmacologically acceptable salt thereof and a pharmacologically acceptable carrier.
